# EUROPEAN PATENT SPECIFICATION

(11) **EP 0 642 471 B1**
(45) Date of publication and mention of the grant of the patent: **10.02.1999**
(21) Application number: 92914334.5
(22) Date of filing: 26.06.1992
(51) Int. Cl.: C02F 3/12, C05F 3/00, A01C 3/02

(54) **METHOD FOR FERMENTATION OF ESPECIALLY HIGHLY VISCOUS SUBSTRATES AND MEANS FOR BLENDING AIR INTO THE SUBSTRATE**
VERFAHREN ZUR GÄRUNG VON BESONDERS ZÄHFLÜSSIGEN SUBSTRATEN UND VORRICHTUNG FÜR DIE EINMISCHUNG VON LUFT IN DAS SUBSTRAT
PROCEDE POUR LA FERMENTATION DE SUBSTRATS PARTICULIEREMENT TRES VISQUEUX ET DISPOSITIF POUR INTRODUIRE DE L'AIR A MELANGER DANS LE SUBSTRAT

(30) Priority: 28.06.1991 SE 9102019
(43) Date of publication of application: 15.03.1995
(73) Proprietor: ALFA-LAVAL AGRICULTURE INTERNATIONAL AB, 147 21 Tumba (SE)
(72) Inventor: SKJELHAUGEN, Odd, Jarle, N-1800 Askim (NO); SAETHER, Trond, N-1990 Sörum (NO); LIDMAN, Magnus, S-144 00 Rönninge (SE)
(74) Representative: Lerwill, John
(86) International application number: SE9200478
(87) International publication number: WO9300302

(56) References cited:
- DE-A- 2 250 827
- DE-A- 2 449 212
- PATENT ABSTRACTS OF JAPAN, Vol. 11, No. 202, C432; & JP,A,62 023 498, publ 1987-01-31 (EBARA INFILCO CO LTD).

## Description

The present invention relates to a method for fermentation of especially highly viscous substrate in a fermentation tank with a controlled mixing of air and an arrangement for mixing air into the substrate.

It is necessary to supply air to the fermentation tank in order to make the microorganisms grow in aerobic fermentations. Such arrangements for supply of air may be designed in many ways. One known arrangement for supply of air consists of a tube coil provided with holes which is arranged close to the bottom of the fermentation tank. The curtain of air bubbles which is formed is then distributed in the tank by means of a mixer of turbine type.

Air addition must take place also when waste liquids are purified biologically. In this connection surface aerators as well as different kinds of submerged aeration arrangements are used. In SE 351 198 there is described an arrangement comprising an impeller and an unperforated tube for addition of gas which tube leads to the vicinity of the impeller boss.

When this kind of aerator is used the liquid flows through a distinct aeration zone, where the air is powerfully mixed with the liquid. The mixture of air and liquid then flows towards the surface. The liquid which passes the aeration zone is saturated with oxygen, but it is only a smaller part of the liquid in the fermentation tank which passes the aeration arrangement. In the tank there are consequently partial volumes which are saturated with oxygen and partial volumes which miss oxygen. It is very energy demanding to dimension the capacity for mixing in such way that the whole volume in the tank is brought to flow past the aeration arrangement.

DE-A-2449212 and DE-B-2250827 also describe arrangements with rotary impellers mounted at the lower ends of tubes, in both cases the foam which collects at surface of the liquid becomes drawn down through the tube with the air for further mixing with the liquid by the action of the impeller. JP-A-62023499 discloses an arrangement in which air is introduced by a diffusion device and the foam produced rises through a draft tube.

In industrial fermentations with uniform cell cultures there are many possibilities to control the fermentation. In spontaneous fermentations or wild fermentations as biological purification of waste water or liquid composting control of the addition of oxygen is one of few possible control methods. The present invention aims to provide a method and apparatus enabling more effective addition of oxygen in a fermentation process.

According to the invention there is proposed a method where the addition of air is controlled which method may be used in industrial fermentations where one desires to control the addition of oxygen to the liquid as well as different kinds of wild fermentations. where one needs to control the fermentation course and/or the formation of by-products.

The proposed method is mainly characterised in that the addition of air to the substrate takes place in two steps. In the first step a small part of the substrate is mixed with a larger part of air in such a way that a foam compnsing air bubbles is formed. In the second step the formed foam is distributed and mixed into the substrate in a mixing zone close to the bottom in the fermentation tank, at which very small air bubbles are obtained. the rising speed of which is so low that the degree of utilization of oxygen in percent of the added amount of oxygen is above 20%.

In a fermentation tank, where the method of the invention issued, air bubbles are distributed evenly in the tank at a certain moment. They all move upwards towards the surface with a varying speed. New air bubbles are supplied at the bottom of the tank all the time. Not only the air bubbles but also the surrounding liquid move upwards towards the surface. Liquid which has reached the surface and has been impoverished of air moves downwards towards the bottom. In this way a self-controlling stable system is obtained. In the zone where the foam formation takes place the conditions are turbulent, but for the rest only a limited mixing effect is needed. Foam consists of a network of thin liquid lamellas which surround small limited volumes of gas. When the foam is formed energy is added to the liquid which corresponds to the surface tension in the newly formed surfaces of liquid on both sides of the foam lamella. According to the invention the foam is formed in one moment and in the second the gas volumes which are present in the foam are distributed in the surrounding substrate. The addition of air may therefore take place with a low energy consumption.

The air bubbles in the formed foam may also be distributed in the substrate by hydromechanical working. Such a hydromechanical working may take place with means, which by way of a shearing action divide the foam in connection with the mixing.

The air bubbles in the foam may alternatively be divided further prior to the mixing in the fermentation tank. A smaller diameter of the foam bubbles demands an increase in the amount of liquid present in the surrounding liquid lamella. A mechanical reduction of the size of the foam bubbles must therefore be combined with an addition of liquid. This takes place most simply by a repeated remixing of already formed foam and liquid.

With such a two-step mixing of air very small air bubbles may be obtained. The diameter of the air bubbles is in the interval between some tenth part of a millimeter up to some millimeters. The diameter of the air bubbles is suitably below one millimeter. Such small air bubbles have a low rising speed which means that they are available for a longer time for the microorganisms in the fermentation tank. The more viscous the substrate is the slower is the rising speed. Many small air bubbles also give smaller average distance between the air bubbles. The contact area between gas-liquid is large, which also results in a high and constant degree of utilization of oxygen.

Foam forming means may if so is desired also be added to the substrate in the fermentation tank.

A device for mixing air into highly viscous substrate according to the method of the invention comprises a tube for addition of air to a rotary foam forming means which, in use is submerged in the substrate in the fermentation tank. The means comprises two elements with confronting surfaces, the surface of at least one of the elements having lowered and/or raised parts. The elements are arranged to be movable in a transversely way in relation to each other a distance from each other corresponding to the thickness of the desired liquid lamella. Whether the surface parts are considered to be lowered or raised depends on the plane defined as the main plane, but the surface form is such that the surface of the raised parts is situated at a small distance equal to The desired thickness of the liquid film from the most adjacent surface of the other element. The spaces which are defined by the lowered parts in the structure are connected to the air pipe and to receive surrounding substrate such that a foam is formed between the elements. Means are arranged to distribute the formed foam in the substrate close to the depth at which the foam forming means is submerged in the fermentation tank, in particular close to the bottom of the tank.

When the foam is formed a liquid is stretched to thin lamellas. If a larger amount of foam of a highly viscous liquid should be produced, an arrangement is needed which continuously creates thin liquid lamellas by mechanically shearing the liquid to a thin film which immediately is exposed to the air or the gas which one desires to surround. According to the invention a suitable design of the surface of at least one of the elements may be achieved in many different ways. The structure may be formed with regular or irregular bars, grooves, rods or tube pieces. The vital thing is that the element has a surface which in cooperation with the confronting surface of the other element may form a thin liquid film when the elements move in relation to each other. The surface structure of the element shall also contain lowered parts intended to contain air in order to facilitate foam forming.

The confronting surfaces of the elements are movable transversely in relation to each other, which means that the relative movement is essentially translational. One of the elements may be movable and the other stationary or both may be movable. The simplest arrangement from a construction view point is with one of the elements stationary. In the slit between the confronting surfaces of the stationary and the moveable elements, the liquid is sheared into a thin film. Since the surface has a form with lowered or raised parts. the liquid layer is exposed to a lowered part, that is a space which is mainly filled with air and earlier formed foam. For each passage a number of foam bubbles consequently is formed.

The simpliest way to create the necessary movement to form foam is based on rotation.

The foam forming means is suitably designed such that it also distributes the formed foam in the substrate. If it is desirable the foam formation may take place at some other place in the fermentation tank.

The formation of foam advantageously takes place between a rotating part and a stationary part. If the rotating part is shaped as a cylindrical part within a stationary housing, the movable or the stationary part or both are provided with grooves or rods with a pitch having a screw form.

The foam forming means is with advantage shaped such that the two elements consist of one stationary and one rotating disc, which both have a structure with recessed and/or raised parts in the shape of radial rods, the space between the discs being connected to the tube for air addition and holes for addition of substrate being arranged in the stationary and/or in the rotating disc. The formed foam is pumped into the surrounding liquid by influence of the rotation.

The foam forming means may have a small diameter, which at a certain speed of the motor gives a high shearing speed. At the same time there is obtained an efficient distribution of foam in the liquid.

The distance between lowered and/or raised parts in the surface structure may be 1-100 mm, preferably 10-30 mm.

When carrying through the method according to the invention the degree of utilization of oxygen in the added air is above 20 %. How well the oxygen may be utilized depends on many factors, for example the shape of the tank, the viscosity of the substrate, the amount of microorganisms and their demands for oxygen. In many processes it is desirable to be able to use the oxygen in an optimal way. When fermentating liquid manure one wants to obtain such a high degree of oxygen utilization as 90-95 % in order to avoid emission of ammonia. When fermentating for production of energy (heat energy) one wants to avoid unnecessarily large amounts of air since the heating and moisturizing of the air which passes the substrate constitute a loss of energy.

The method according to the invention is suitable for example for a fermentation where the substrate consists of liquid manure with a high content of dispersed material. By adding the air in the form of finely divided air bubbles the fermentation may be carried out with controlled aerobic conditions, which results in an retardation of the formation of ammonia such that the emission of ammonia to the surrounding atmosphere may be kept low.

The manure which is obtained when holding animals must be treated in order not to be considered as a waste problem. Manure in solid form has since long been composted. Due to the rise of temperature to 40-60° C which takes place during the composting the amount of pathogenic bacterias, parasites and weeds is diminished. The smell is also made more agreeable. Liquid manure may also be composted, but the composting demands addition of air to the liquid.

Both when composting solid and liquid manure there is problem with losses of ammonia with the departing air. Earlier the ammonia emission has been considered as mainly a loss of nitrogen. With the increased knowledge of the dangerous effect on the environment of the ammonia it is necessary to lower the emission of ammonia. This has taken place by filtering the leaving air which has been brought to pass peat beds or acid water solutions. Attempt has been made to add clay or to increase the amount of straw in order to increase the amount of degradable carbohydrates, which should result in an increased concentration of carbonate and hydrogen carbonate ions. Attempt has also been made to switch between aerobic and anaerobic fermentation by varying the amount of added air. During the aerobic fermentation an increase in pH and in formation of ammonia are obtained, while the anaerobic fermentation results in a lowering of the pH and binding of the ammonia.

None of the methods described above has worked well in practice.

By using the method according to the invention the emission of ammonia to the environment may be reduced considerably. One possible explanation of this effect is that a large part of the nitrogen in the fresh manure is bound as urea. Urea is decomposed by the enzyme ureas to ammonia and carbonate ions. At raised pH values the decomposition takes place by strictly aerobic microorganisms which demand a good access of oxygen. If the oxygen concentration is limited the decomposition is hindered. According to the present invention there are air bubbles distributed in the whole volume of liquid. The oxygen from the air bubbles is dissolved into the liquid to a constant but low level.

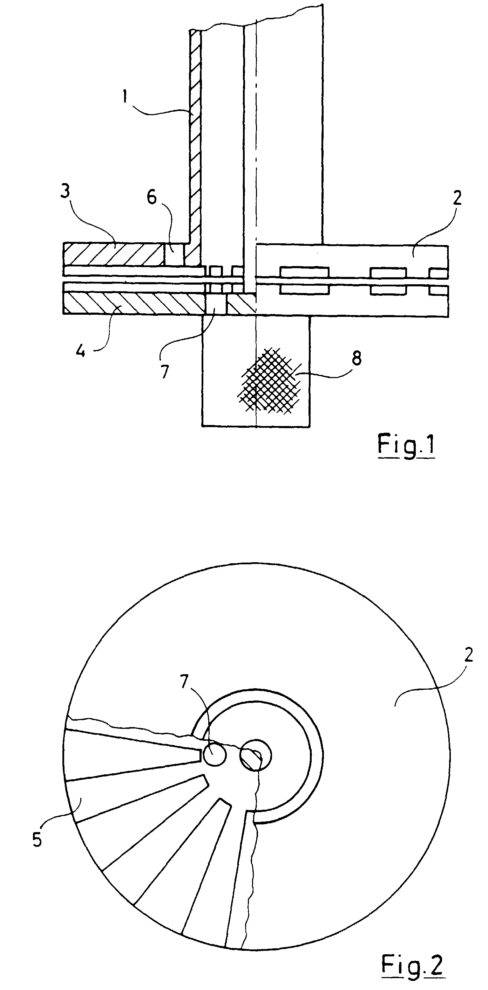

An arrangement for mixing air according to the invention is described in the attached drawing, fig 1 of which shows the foam forming means seen partly in section, partly from the side and fig 2 of which shows a section between the both discs, as well as the disc 2 seen from above.

The arrangement comprises a tube 1 for supply of air to a foam forming means 2 which is submerged in the substrate in a fermentation tank (not shown). An upper stationary disc 3 is connected to the tube 2 for addition of air. Under the stationary disc 3 there is a rotating disc 4. Both the stationary and the rotating disc have radial grooves 5 (fig 2) formed between raised rods or ribs. In the form which is shown in figure 1 and 2 both the stationary and the movable disc have holes for addition of substrate. If the substrate contains large amounts of suspended material for example plant fibres, the means is designed with holes only in the rotating disc. The holes are suitably formed such that they are self cleaning.

The rotating disc may be provided with a sieve arrangement 8 which also rotates. The proportions between air and liquid in the foam (foam density) is controlled by the air flow through the pipe and the liquid flow through the holes together with the characteristics of the pump wheel of the discs 2 and 3 (peripheral speed and the design of the flow channels).

The pore size of the foam and consequently the size of the air bubbles in the fermentation tank is controlled by the slit height between the stationary and the rotating disc and of the shearing speed of the liquid between the discs.

The example given below describes how the method according to the invention is carried out when composting liquid manure. The fermentation tank has a volume of 10 m³ with a cylindrical form and with a bottom in the form of a frustum of a cone. The diameter was 2,2 m. From the start the tank was an insulated but during the series of trials it was shown that insulation was demanded. For comparison first trials were carried out with prior art air mixers having impellers and sold under the names Aldo 100 4kW and Biojet 2,2 kW. The latter gives a better result than the first but the result was still unsatisfactory.

Data for two fermentations of liquid manure carried through according to the method of the invention are given below.

| | Trial 1 | Trial 2 |
|---|---|---|
| Time | 5 days Nov -90 | 9 days Feb -91 |
| Reaction temperature | 45-52° C | 43-50° C |
| Temp. distribution in the tank | +1-0° C | +1-0° C |
| Added amount of air | 3,1-2,4 kg/H | 4,7-3,2 kg/h |
| Added amount of oxygen | 0,6-0,5 kg O₂/h | - |
| Utilization of oxygen | 90-100 % | 80-90 % |
| Addition of energy W/m³ volume of the reactor | 140 | 140 |
| Loss of ammonia | 0,3 g/h | 0,5 g/h |
| pH-value in the liquid | 7,9-8,1 | 8,1-8,3 |
| Dissolved oxygen in the liquid | 0,5-0,8 ppm | 0,2-0,6 ppm |
| Energy consumption kWh/m³ for treated liquid | 16 | 16 |

By directing the leaving air containing ammonia to pass a heat exchanger where it is cooled by air which is sucked into the tank the emission of ammonia may be lowered further. Condensed water comprising ammonia ions is then returned to the tank.

In liquid manure from cattle the viscosity may be as high as 2000 cP. As a mean value during a composting process a value of the viscosity ∼ 300 cP may be used. An air bubble of a diameter of 1 mm then has a rising speed of 1,82 mm/sek (Navier-Stok). If the composting tank contains an amount of liquid with a height of 2 m the mean holding time is around 18 min, if the movement of the bubble only depends on differences in density. Such a long contact time makes the utilization degree of oxygen high. The volume air which is mixed into the substrate may therefore be relatively small. Only 15-20 % of the air flow which earlier used air mixers added, is sufficient to give a satisfactory content of air in the liquid. Even if the power requirement per m³ mixed air increases when the size of the bubbles is smaller, the net power may be low due to the reduction of the air flow.

A detailed description has been made above of a specific application of the method according to the invention. Another application is fermentations where one desires to control or influence the fermentation as for example when utilizing reaction heat from aerobic degradation.

The necessary amounts of nitrogen, potassium and phosphor are then added in the form of liquid manure, latrine or industrial waste. The amount of carbohydrate which is needed for energy production is added in the form of fibrous waste or plant material. A substrate with a high content of energy per volume unit has inferior liquid properties. It is difficult to carry out an addition of oxygen such that the degree of utilization of oxygen is high and the losses of heat to air flowing through the liquid are kept low.

The method according to the invention may with advantage also be used when the fermentation results in products with a high viscosity.

## Claims

1. Method for fermentation of substrate in a fermentation tank especially highly viscous substrate, comprising a controlled addition of air, characterised in that the addition of air takes place in two steps, where a small amount of substrate is mixed with a larger amount of air in the first step in such a way that a foam containing air bubbles is formed, which foam in the second step is distributed and mixed into the substrate, both the first and the second step of addition of air are carried out in a mixing zone close to the bottom of the fermentation tank, and very small air bubbles are formed and have a rising speed so low that the degree of utilization of the oxygen in percent of the added amount of oxygen is above 20%.

2. Method according to claim 1, characterised in that the air bubbles which are obtained during the foam formation are distributed by hydromechanical working in the mixing zone.

3. Method according to claim 1, characterised in that air bubbles which are obtained at the foam formation are divided further by repeated remixing of foam and substrate.

4. Arrangement for mixing air into a substrate, especially a highly viscous substrate, by the method for fermentation according to claim 1, characterised in that it comprises a tube (1) for supply of air to a rotary foam forming means (2) which, in use, is submerged in the substrate in a fermentation tank, which means comprises two elements (3,4) with confronting surfaces, the surface of at least one of the elements having raised and/or lowered parts and the surfaces being arranged to be movable transversely in relation to each other and at a distance from each other corresponding to the size of the desired liquid lamella, the spaces defined by lowered parts of the surface of said at least one element are connected to the air tube and to receive surrounding substrate in such a way that the foam is formed between the elements, and means are arranged to distribute the formed foam into the substrate close to the depth at which the foam forming means is submerged in the fermentation tank.

5. Arrangement according to claim 4, characterised in that the foam forming means is designed also to distribute the formed foam in the substrate.

6. Arrangement according to claim 5, characterised in that the two elements (3,4) of the foam forming means comprise a stationary disc (3) and a rotatable disc (4) both discs have surfaces with radial grooves (5), the space between the discs being connected to the air supply tube (1), and holes (6,7) for admission of substrate are arranged in the stationary and/or in the rotatable disc.

## Patentansprüche

1. Verfahren zur Gärung eines Substrates in einem Gärungstank, insbesondere für ein besonders zähflüssiges Substrat, unter gesteuerter Zufuhr von Luft, **dadurch gekennzeichnet, daß** die Luftzufuhr in zwei Schritten erfolgt, wobei
in einem ersten Schritt eine kleine Menge des Substrates auf eine solche Weise mit einer größeren Menge von Luft gemischt wird, daß ein Schaum gebildet wird, der Luftblasen enthält und der
im zweiten Schritt verteilt und in das Substrat gemischt wird, wobei
der erste und der zweite Schritt der Luftzufuhr in einem Mischbereich ausgeführt werden, der sich nahe dem Tankboden befindet und wobei
sehr kleine Luftblasen gebildet werden, die eine so geringe Aufstiegsgeschwindigkeit haben, daß der Grad der Ausnutzung des Sauerstoffs über 20 % der hinzugefügten Sauerstoffmenge beträgt.

2. Verfahren nach Anspruch 1, **dadurch gekennzeichnet, daß** die Luftblasen, die während der Schaumbildung entstehen, durch hydromechanisches Verarbeiten im Mischbereich verteilt werden.

3. Verfahren nach Anspruch 1, **dadurch gekennzeichnet, daß** die Luftblasen, die während der Schaumbildung entstehen, weiterhin geteilt werden durch wiederholtes Wiedervermischen von Schaum und Substrat.

4. Anordnung zur Einmischung von Luft in ein Substrat, insbesondere ein besonders zähflüssiges Substrat, nach dem Gärungsverfahren nach Anspruch 1, **dadurch gekennzeichnet, daß** sie eine Röhre (1) für die Zufuhr von Luft an eine rotierende Schaumbildungseinrichtung (2) umfaßt, die bei ihrer Verwendung in das Substrat im Gärungstank eingetaucht wird, wobei diese Einrichtung zwei Elemente (3, 4) mit gegenüberstehenden Oberflächen umfaßt, wobei
mindestens eins der Elemente angehobene und/oder abgesenkte Teile aufweist;
die Oberflächen so angeordnet sind, daß sie transversal in Bezug zueinander und in einem Abstand voneinander beweglich sind, der der Größe der gewünschten Flüssigkeitslamelle entspricht;
die Abstände, die durch die abgesenkten Teile der Oberfläche von mindestens einem Element gebildet werden, mit der Luftröhre verbunden sind und das umgebende Substrat in solcher Weise aufnehmen, daß der Schaum zwischen den Elementen gebildet wird, und
Einrichtungen vorgesehen sind, die so angeordnet sind, daß der gebildete Schaum in der Nähe der Tiefe, wo die Schaumbildungseinrichtung in den Gärungstank eingetaucht ist, im Substrat verteilt wird.

5. Anordnung nach Anspruch 4, **dadurch gekennzeichnet, daß** die Schaumbildungseinrichtung so ausgestaltet ist, daß auch der gebildete Schaum im Substrat verteilt wird.

6. Anordnung nach Anspruch 5, **dadurch gekennzeichnet, daß** die zwei Elemente (3, 4) der Schaumbildungseinrichtung eine feststehende Scheibe (3) umfassen und eine drehbare Scheibe (4), wobei beide Scheiben Oberflächen mit radialen Rillen (5) aufweisen, wobei der Abstand zwischen den Scheiben mit dem Luftzufuhrrohr (1) verbunden ist, und wobei sich Löcher (6, 7) für die Zufuhr des Substrats in der feststehenden und/oder drehbaren Scheibe befinden.

## Revendications

1. Procédé pour la fermentation de substrat dans un bassin de fermentation, en particulier d'un substrat extrêmement visqueux, comprenant une addition contrôlée d'air, caractérisé en ce que l'addition d'air s'effectue en deux étapes, avec une petite quantité de substrat mélangé avec une plus grande quantité d'air dans une première étape afin de former une mousse contenant des bulles d'air, laquelle mousse dans la seconde étape est distribuée et mélangée dans le substrat, la première et la seconde étape d'addition d'air étant toutes deux effectuées dans une zone de mélange proche du réservoir de fermentation et de très petites bulles d'air sont formées et ont une vitesse ascendante d'une lenteur telle que le degré d'utilisation de l'oxygène en pourcentage de la quantité ajoutée d'oxygène est supérieur à 20 %.

2. Procédé selon la revendication 1, caractérisé en ce que les bulles d'air qui sont obtenues pendant la formation de mousse sont réparties par travail hydromécanique dans la zone de mélange.

3. Procédé selon la revendication 1, caractérisé en ce que les bulles d'air qui sont obtenues lors de la formation de mousse sont encore divisées par remélange répété de mousse et de substrat.

4. Agencement pour mélanger de l'air en un substrat, notamment un substrat extrêmement visqueux, par le procédé de fermentation selon la revendication 1, caractérisé en qu'il comprend un tube (1) pour l'amenée de l'air sur des moyens de formation de mousse rotative (2) qui, dans l'utilisation, sont immergés dans le substrat dans le réservoir de fermentation, moyens qui comprennent deux éléments (3,4) avec des surfaces en confrontation, la surface d'au moins l'un des éléments présentant des parties relevées et/ou abaissées et les surfaces étant disposées de façon à pouvoir se déplacer transversalement l'une par rapport à l'autre et à une distance l'une de l'autre correspondant à la taille des lamelles de liquide souhaité, les espaces définis par les parties abaissées de la surface d'au moins un élément, sont raccordés au tube d'air de façon à recevoir le substrat avoisinant, de sorte que la mousse est formée entre les éléments et les moyens sont disposés de façon à répartir la mousse formée dans le substrat à proximité de la profondeur à laquelle le moyen formant la mousse est immergé dans le réservoir de fermentation.

5. Agencement selon la revendication 4, caractérisé en ce que les moyens formant la mousse sont conçus également pour répartir la mousse formée dans le substrat.

6. Agencement selon la revendication 5, caractérisé en ce que les deux éléments (3,4) des moyens formant la mousse, comprennent un disque stationnaire (3) et un disque rotatif (4), les deux disques ayant des surfaces avec des gorges radiales (5), l'espace entre les disques étant raccordé au tube d'alimentation (1), et des trous (6,7) pour l'admission du substrat sont disposés dans le disque stationnaire et/ou rotatif.
